# FASCICULE DE BREVET EUROPEEN

(11) **EP 2 571 468 B1**
(45) Date de publication et mention de la délivrance du brevet: **23.04.2014**
(21) Numéro de dépôt: 11727212.0
(22) Date de dépôt: 16.05.2011
(51) Int. Cl.: A61F 13/02, A61F 13/06

(54) **COMPLEXE DE TRAITEMENT, NOTAMMENT ORTHÈSE**
BEHANDLUNGSKOMPLEX, IM BESONDEREN EINE ORTHESE
TREATMENT COMPLEX, IN PARTICULAR AN ORTHOSIS

(30) Priorité: 20.05.2010 FR 1002135
(43) Date de publication de la demande: 27.03.2013
(73) Titulaire: Rebuffet, Michel, 38410 Saint Martin d'Uriage (FR)
(72) Inventeur: Rebuffet, Michel, 38410 Saint Martin d'Uriage (FR)
(74) Mandataire: Prugneau, Philippe
(86) Numéro de dépôt international: PCT/FR2011/051090
(87) Numéro de publication internationale: WO 2011/144853

(56) Documents cités:
- WO-A1-00/49982
- WO-A1-98/02120
- US-A- 5 016 621
- US-A- 5 538 500
- US-A- 5 865 776
- US-A1- 2004 260 224
- US-A1- 2005 049 537
- US-A1- 2005 283 106

## Description

### Domaine technique

La présente invention concerne un complexe de traitement pour zone anatomique du corps humain, en particulier une orthèse pour genou, cheville, épaule, coude ou poignet, un pansement ou un timbre transdermique.

### Technique antérieure

Les orthèses sont couramment utilisées pour servir d'assistance articulaire ou musculaire ou pour la stabilisation d'une partie du corps, le plus souvent une articulation. Les orthèses peuvent être utilisées selon différentes prescriptions telles que par exemple : immobilisation, mobilisation statique, mobilisation dynamique, restriction de mouvements, compression d'une zone anatomique. Dans la suite, on appelle « traitement » toutes ces configurations d'utilisation. Il existe de nombreux types d'orthèses réalisées en textile et/ou en matériaux plastiques. Pour atteindre un meilleur confort et un maintien amélioré, certaines orthèses sont en partie réalisées en silicone.

Le Silicone ou polysiloxane, est un polymère inorganique formé d'une chaîne principale composée d'atomes de Silicium et d'Oxygène alternés. Les chaînes principales sont reliées entre-elles par des groupes organiques. Ainsi, en faisant varier les chaînes, les groupes organiques et les liaisons, on obtient une grande variété de silicones différents, de consistance variée pouvant aller du liquide au plastique dur en passant par le gel et la gomme. Suivant leurs états physiques (viscosité, pourcentage de réticulation, propriétés mécaniques), les silicones sont regroupés en trois grandes familles : les fluides, les résines et les élastomères. De plus, différentes charges et additifs peuvent être ajoutés pour obtenir les propriétés désirées. Les élastomères de silicone sont très largement utilisés notamment sous forme de mastics, colles, joints, additifs anti-moussant pour poudres lessivielles, cosmétiques, matériel médical, gaines isolantes de câbles électriques, graisses haute performance, ... Les élastomères de silicone sont ainsi utilisés dans des domaines très variés tels que notamment la bureautique, l'industrie textile, l'industrie alimentaire, le bâtiment, les transports, l'électricité, l'électronique, la cosmétique et le médical. Les élastomères de silicone sont généralement classés en fonction de la forme de vulcanisation employée pour les mettre en oeuvre à savoir une vulcanisation à froid ou une vulcanisation à chaud.

Des exemples d'orthèses réalisées en partie en silicone sont notamment donnés dans les publications citées ci-après. La publication US 4,442,833 décrit une orthèse pour poignet comportant une couche résiliente en mousse de silicone destinée à être en contact avec le poignet et prévue sur un support textile lui-même imprégné d'une résine de silicone. Les publications WO 88/00 819 et US 4,700,698 décrivent chacune une orthèse de genou comportant un anneau en silicone destiné à entourer la rotule du genou et assurer son orientation. La publication US 2007/010 773 décrit une orthèse de cheville comportant une structure élastique continue formant un tube et pourvue, au niveau du coup de pied, d'un renfort en silicone. Le silicone présente de bonnes caractéristiques d'amortissement et de confort permettant de recommander son utilisation pour ce genre d'application. Il est également utilisé pour sa faible énergie de surface qui lui confère des propriétés antiadhésives. En effet, de manière générale, les élastomères de silicone utilisés présentent une composante dispersive unique de l'énergie de surface de l'ordre de 18 - 22 mJ/m². L'adhérence du silicone est donc quasiment nulle sur pratiquement toutes les surfaces avec lesquelles il est mis en contact, en particulier avec la peau. Ainsi, les orthèses comportant du silicone peuvent être aisément enfilées sur le membre ou sur le tronc, sans pour autant tirer la peau. De plus, le silicone est non irritant et non sensibilisant pour la peau.

Malgré leurs avantages, les orthèses connues présentent un inconvénient majeur. En effet, ces orthèses ont tendance à glisser sur la peau. Aussi, pour avoir une action efficace, leur position doit être fréquemment réajustée et les orthèses doivent être portées très serrées. Par ailleurs, la morphologie étant très différente d'une personne à une autre, il est nécessaire de prévoir un nombre important de tailles d'orthèse pour s'adapter à chaque morphologie.

On connait par ailleurs, de la publication US 5,865,779, une orthèse pour genou se présentant sous la forme d'un fourreau élastique dont les extrémités sont pourvues de bande velcro permettant le resserrement de l'orthèse autour du genou. Ce fourreau est pourvu de pans de serrages différenciés et est réalisé dans un matériau en trois épaisseurs comportant une épaisseur intermédiaire, formée d'un film en polyuréthane élastique, entourée de faces externes en étoffe à base de 80% de nylon et 20% de Spandex®. Cette orthèse comporte une ouverture apte à recevoir l'extrémité de la rotule et un logement en U, longeant cette ouverture, ce logement étant apte à recevoir un raidisseur réalisé en silicone, amovible. La face intérieure de l'extrémité supérieure du fourreau est pourvue d'une bande antidérapante facilitant la mise en place de l'orthèse et son maintien en position pendant l'exercice physique. Cette bande antidérapante est réalisée à base d'une résine silicone (Ipocon) appliquée directement sur le fourreau en une couche fine au moyen d'une brosse, puis cuite pendant au moins 24 heures.

On connait également, de la publication US 2005/ 049 537, un bandage thérapeutique comportant une bande élastique réalisée en non-tissé. Selon un mode de réalisation illustré, les extrémités latérales de la bande élastique sont aptes à se fixer l'une sur l'autre après enroulement du bandage thérapeutique autour du membre concerné. De plus, le bandage thérapeutique comporte une zone adhésive centrale prévue entre les extrémités latérales et réalisée en silicone ou hydrogel. Cette zone adhésive étant destinée à venir en contact avec la peau et peut comporter des additifs thérapeutiques tels que notamment des plantes, des vitamines, ...

On connait également, de la publication WO 98/02 120, un bandage antidérapant comportant une bande réalisée en matériau textile et une languette de fermeture de type velcro. La bande sert de support à des points ou lignes adhésifs, par exemple en silicone.

On connait également, de la publication US 2004/0 260 224, un bandage pour suspendre un bras comportant une bande en matériau étirable tel que du Velstretch® dont la face intérieure est recouverte d'un gel, par exemple un gel de silicone, destiné à adhérer sur la peau et à empêcher le glissement du bras en dehors du bandage et garantir ainsi le maintien efficace du bras.

On connait également, de la publication WO 00/49 982, un fourreau de compression comportant une bande en matériau textile dont la face interne est pourvue de picots en silicone anti-glissement.

Les orthèses et bandage décrits dans ces documents, ne permettent toutefois pas d'apporter une réponse satisfaisante tant pour le maintien efficace de l'orthèse ou bandage lors de l'utilisation que pour le nombre important de tailles d'orthèse ou bandage nécessaire pour les morphologies variées.

Les pansements et les timbres transdermiques sont utilisés dans différentes configurations que l'on appelle dans la suite « traitement ». Les pansements sont des complexes de traitement utilisés par exemple pour l'aide à la cicatrisation (brulure, ampoule, ...), la protection d'une zone de peau contre certaines agressions extérieures (frottement, UV, ...) ou toute autre application similaire. Ils se présentent généralement sous la forme d'un matelas de protection souple, prolongé latéralement (ou sur toute sa circonférence) par une zone adhésive permettant la fixation du pansement sur la peau. Les timbres transdermiques comportent des complexes de traitement souples et adhésifs comportant une substance active telle que par exemple des oestrogènes, de la nicotine, de la Lidocaïne, recouvert généralement sur toute leur surface d'une couche adhésive permettant au timbre transdermique d'adhérer à la peau. Les timbres transdermiques sont utilisés pour l'administration par voix transcutanée de la substance active. Ils permettent d'assurer une libération lente et contrôlée de la substance active dans le corps via le sang ou la lymphe grâce à la chaleur corporelle et à la solubilité de la substance active. La face externe des timbres transdermiques est fréquemment recouverte d'une couche imperméable autorisant la prise de douches sans endommager les timbres transdermiques.

Néanmoins, l'adhésion des pansements et les timbres transdermiques est souvent relevée comme étant un point faible important. De plus, une fois décollés, ces pansements et timbres transdermiques ne sont plus réutilisables, leur adhérence est insuffisante pour autoriser leur maintien sur la peau.

### Exposé de l'invention

La présente invention vise à remédier à ces inconvénients en proposant un complexe de traitement pour zone anatomique, notamment une orthèse, simple à mettre en place, réutilisable, confortable, ayant un bon maintien, assurant un traitement efficace et, en particulier une orthèse, conférant une contention adaptée tout en autorisant la mobilisation souhaitée de la zone anatomique, limitant le nombre de tailles nécessaires pour s'adapter aux différentes morphologies.

L'invention concerne un complexe de traitement pour zone anatomique du corps humain, ledit complexe comportant au moins une zone active pour traiter ladite zone anatomique, ladite zone active étant couplée à des moyens principaux d'accrochage prévus de part et d'autre de ladite zone active et aptes à solidariser ledit complexe à ladite zone anatomique, les bords latéraux dudit complexe de traitement étant disjoints et non reliés entre eux au moins lorsque ledit complexe de traitement n'est pas porté, caractérisé en ce que ladite zone active est au moins en partie réalisée dans un premier silicone, en ce que ledit complexe de traitement comporte des moyens secondaires d'accrochage, distincts desdits moyens principaux d'accrochage, disposés entre lesdits moyens principaux d'accrochage et couplés à ladite zone active, lesdits moyens secondaires d'accrochage comportant au moins une première zone d'adhérence réalisée au moins en partie dans un second silicone ayant des caractéristiques d'adhésion à la peau supérieures à celles dudit premier silicone, ladite première zone d'adhérence étant apte à coopérer avec la peau de ladite zone anatomique pour au moins contribuer à l'adhésion de ladite zone active sur ladite peau.

La zone active réalisée en silicone est ainsi élastiquement déformable. Le complexe de traitement peut ainsi s'adapter à différentes morphologie et en épouser facilement les reliefs. Les moyens secondaires d'accrochage permettent au complexe de traitement d'adhérer à la zone anatomique avant d'être fixé dessus par les moyens principaux d'accrochage. Le complexe de traitement est ainsi simple à mettre en place. De plus, les moyens secondaires d'accrochage permettent au complexe de traitement d'avoir un pouvoir d'adhérence au niveau de la zone active fiabilisant le maintien du complexe de traitement pendant son port. De plus, les différents niveaux et formes d'adhérence peuvent être dissociés et adaptés notamment aux sollicitations afin d'améliorer le confort du complexe de traitement et de garantir un maintien et un traitement efficaces.

Ladite première zone d'adhérence comporte avantageusement au moins un coussinet ayant une forme bombée, définissant par-rapport à ladite zone active un premier relief.

Selon un mode de réalisation avantageux, ladite première zone d'adhérence comporte une pluralité de coussinets en silicone ayant chacun une forme bombée, définissant par rapport à ladite zone active des premiers reliefs distants les uns des autres agencés pour ménager entre eux un réseau libre apte à autoriser la circulation de l'air entre ladite zone anatomique et ladite zone active lorsque ledit complexe de traitement est en appui contre ladite zone anatomique. Le confort, lors du port du complexe de traitement est largement amélioré par cette circulation de l'air qui limite les effets de la sudation.

Ladite zone active est de préférence pourvue d'un orifice apte à accueillir une partie de la zone anatomique et ladite première zone d'adhérence comporte de préférence un anneau de maintien en silicone entourant ledit orifice, ledit anneau de maintien définissant par rapport à ladite zone active un premier relief.

Ledit orifice présente avantageusement des dimensions inférieures aux dimensions intérieures dudit anneau de maintien de manière à définir une lèvre d'appui entourée dudit anneau de maintien et entourant ledit orifice, ladite lèvre d'appui étant apte à servir de support à un anneau amovible logé dans ledit anneau de maintien.

Cette lèvre d'appui peut être prolongée par un retour orienté vers le coté de l'orthèse comportant ledit anneau de maintien ledit retour définissant, avec ladite lèvre d'appui et ledit anneau de maintien, une gorge apte à recevoir ledit anneau amovible et à participer à son maintien

De manière préférée, ledit complexe de traitement est pourvu d'au moins un élément actif comportant au moins une substance active apte à se diffuser vers ladite peau pour assurer l'administration par voie transcutanée de ladite substance active.

De manière préférée, ledit complexe de traitement comporte au moins un anneau amovible comportant au moins une substance active et formant ledit élément actif, ledit anneau amovible étant inséré entre ladite lèvre d'appui et ledit anneau de maintien.

Selon un mode de réalisation préféré au moins ladite zone active est exempte de matériau textile. C'est de préférence l'orthèse entière qui est exempte de matériau textile. De manière avantageuse, l'orthèse n'est réalisée qu'en silicones.

Ladite zone active est avantageusement prolongée latéralement par au moins deux ailes d'accrochage prévues de part et d'autre de ladite zone active, chaque aile d'accrochage étant pourvue d'une zone d'accrochage mâle/femelle apte à coopérer avec une zone d'accrochage femelle/mâle complémentaire et définissant au moins en partie lesdits moyens principaux d'accrochage.

De manière avantageuse, ledit premier silicone a une dureté inférieure à 1 Shore A et de préférence sensiblement égale à 55 type 000 shore A.

Ledit second silicone a de préférence un allongement à la rupture supérieur à 600 % et de préférence d'au moins 670 %.

De manière avantageuse, ledit second silicone a une dureté comprise entre 20 et 60 Shore A et de préférence comprise entre 30 et 50 Shore A.

Ledit second silicone a de préférence un allongement à la rupture supérieur à 520 %, de préférence supérieur à 600 %, et une force à 200 % d'allongement d'au moins 2 MPa et de préférence d'au moins 2.5 MPa.

L'invention concerne également une orthèse pour zone anatomique du corps humain, ladite orthèse comportant au moins une bande de tension apte à être déformée élastiquement, tendue et appliquée sur ladite zone anatomique pour assurer son maintien, caractérisée en ce qu'elle se présente sous la forme d'un complexe de traitement tel que décrit précédemment, ladite orthèse comportant une zone médiane définissant au moins en partie ladite zone active et pourvue sur une de ses surfaces d'au moins une partie desdits moyens secondaires d'accrochage.

L'orthèse est de préférence formée par au moins deux pans de maintien juxtaposés et reliés entre eux, au niveau de ladite zone médiane, par une zone de jonction délimitant les bords latéraux de ladite partie médiane de ladite orthèse, ladite zone de jonction ayant une largeur inférieure à celle desdits pans de maintien. Les pans de maintien peuvent être disjoints et reliés entre eux seulement par une zone de jonction formée par un autre élément. Les pans de maintien peuvent également être reliés directement entre eux en supplément de la liaison formée par un élément supplémentaire.

De manière avantageuse, au moins un desdits pans de maintien comporte au moins une armature, au moins un évidement entouré par ladite armature et un voile en silicone recouvrant ledit évidement.

De préférence au moins un desdits pans de maintien est pourvu d'au moins un bourrelet en relief apte à être plaqué contre ladite zone anatomique lorsque ladite orthèse est portée et formant ledit coussinet, ledit bourrelet étant agencé pour délimiter une zone au moins en partie fermée apte à entourer au moins une partie de ladite zone anatomique.

L'un au moins desdits armature, bourrelet, définit de préférence une forme en huit délimitant au moins deux zones fermées destinées à être placées en regard de ladite zone anatomique et reliées auxdits moyens principaux d'accrochage. Cette forme en huit peut être continue ou discontinue.

L'orthèse peut comporter au moins deux paires de zones de renfort, distantes et décalées latéralement par rapport au centre de ladite zone médiane, chaque zone de renfort ayant une rigidité supérieure à celle de ladite zone médiane, les zones de renfort d'une même paire étant disposées en regard l'une de l'autre et reliées entre-elles par au moins un élément raidisseur élastiquement déformable et ayant une rigidité supérieure à celle de ladite zone médiane.

Chaque zone de renfort comporte avantageusement au moins un fourreau apte à recevoir une extrémité dudit élément raidisseur, lesdits fourreaux des zones de renfort constituant une paire, étant prévus en regard l'un de l'autre, ledit élément raidisseur ayant une longueur inférieure à la distance séparant les extrémités opposées desdits fourreaux l'autorisant à coulisser entre lesdites extrémités pour suivre la déformation angulaire de ladite orthèse, sans dépasser desdits fourreaux.

### Description sommaire des dessins

L'invention est décrite ci-après, à titre d'exemple non limitatif, en référence aux dessins annexés sur lesquels :
- Les figures 1A et 1 B sont des vues respectivement en élévation et de côté d'une orthèse selon un premier mode de réalisation de l'invention ;
- Les figures 2A et 2B sont sensiblement similaires aux figures précédentes et illustrent une orthèse selon un second mode de réalisation d'une orthèse de l'invention ;
- Les figures 3A et 3B sont sensiblement similaires aux précédentes et illustrent une orthèse selon un troisième mode de réalisation d'une orthèse de l'invention
- La figure 4A est sensiblement similaire aux figures 1A-3A et illustre une orthèse selon un quatrième mode de réalisation de l'invention ;
- La figure 4B est une vue partielle en coupe de l'orthèse de la figure 4A selon le plan de coupe AA de la figure 4A ;
- Les figures 5A et 5B sont sensiblement similaires aux figures 4A et 4B et illustrent un cinquième mode de réalisation de l'orthèse selon l'invention, la coupe de la figure 5B étant obtenue selon le plan de coupe BB de la figure 5A ;
- La figure 5C est une vue partielle en coupe de l'orthèse de la figure 5A selon le plan de coupe CC de la figure 5A ;
- Les figures 6A et 6B sont des vues en perspective respectivement de face et de coté d'une orthèse selon un sixième mode de réalisation de l'invention portée, l'orthèse étant représentée portée ;
- La figure 7 est une vue en coupe de l'orthèse des figures 6A et 6B selon le plan de coupe DD de la figure 6A ;
- La figure 8 est une vue partielle arrière de l'orthèse des figures 6A, 6B et 7, cette figure illustrant la partie intérieure de l'orthèse.

Pour des raisons de clarté, la suite de la description est axée sur différents modes de réalisation d'orthèses de genou appelées communément genouillères. Il est bien entendu que l'invention porte sur tout autre type d'orthèse adaptée à toute autre zone anatomique. Les dimensions et la mise en place de l'orthèse seront bien entendu adaptées sans difficulté particulière, par l'Homme du métier, en fonction de la destination de l'orthèse. On utilise donc dans la suite, indifféremment et de manière non limitative, le terme orthèse ou genouillère. L'invention concernée n'est bien entendu pas limitée aux orthèses et porte sur tout type de complexe de traitement pour zone anatomique tel que notamment un pansement, un timbre transdermique.

### Description des modes de réalisation

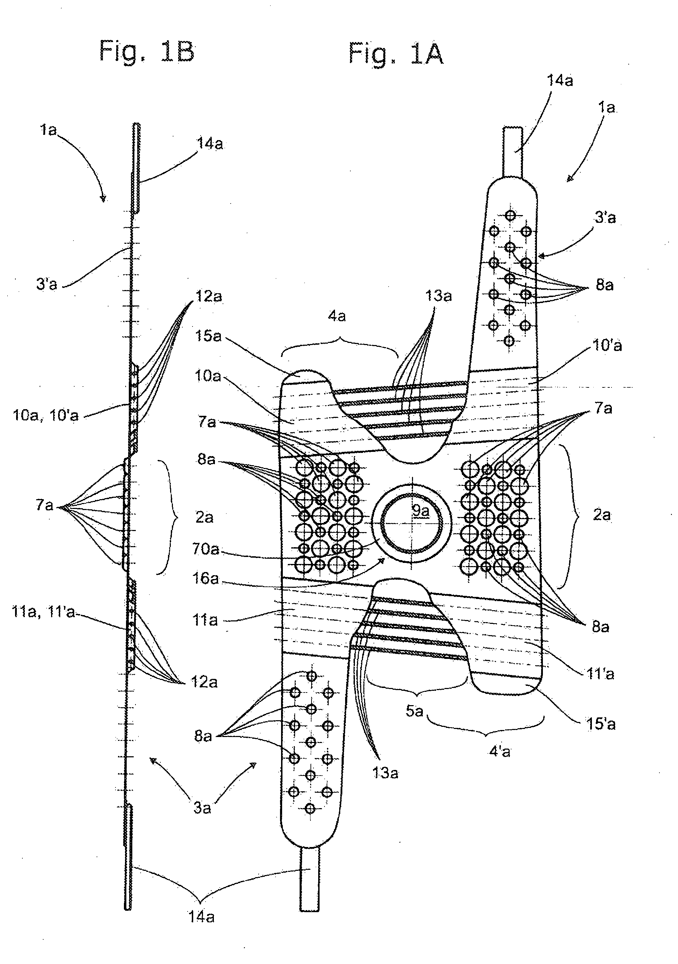

En référence aux figures 1A à 7, les orthèses 1a-1f selon l'invention comportent chacune une bande de tension comportant une zone médiane 2a-f prolongée latéralement par des ailes d'accrochage 3a,3'a-3f,3'f de hauteur inférieure à celle du reste de la bande de tension. L'orthèse 1a-1f a globalement une forme en S, avec une aile d'accrochage supérieure gauche 3'a-3'f et une aile d'accrochage inférieure droite 3a-3f. Elle peut bien entendu avoir une orientation inverse et comporter une aile d'accrochage haute droite et une aile d'accrochage basse gauche. La bande de tension délimite un pan de maintien supérieur 4'a-4'f et un pan de maintien inférieur 4a-4f reliés par une zone de jonction 5a-5f correspondant à la zone centrale de la zone médiane de l'orthèse 1a-1f. Les pans de maintien supérieurs 4'a-4'f et inférieurs 4a-4f sont ainsi juxtaposés et ont une forme sensiblement symétrique par rapport au centre de l'orthèse 1a-1f. La zone de jonction 5a-5f a une largeur inférieure à celle des ailes d'accrochage 3a,3'a-3f,3'f. Selon une variante de réalisation non représentée, la bande de tension peut avoir toute autre forme que celle d'un S. Elle peut notamment comporter une paire d'ailes d'accrochage droites et/ou une paire d'ailes d'accrochage gauches. L'orthèse peut également comporter une bande d'accrochage unique. La ou les ailes d'accrochage peuvent également avoir une hauteur sensiblement égale à celle de la bande de tension.

De manière avantageuse, l'orthèse 1a-1f est construite de manière à ce que, lorsqu'elle n'est pas portée, ses bords latéraux extrêmes ainsi que les bords latéraux de sa zone médiane sont disjoints et non reliés entre eux. Comme décrit plus loin, l'orthèse 1a-1f peut ainsi être mise en place sans devoir être enfilée par le pied mais elle peut être présentée frontalement et ajustée sur le genou sans générer de surépaisseur ce qui est le cas lorsque les bords sont reliés de manière à former un fourreau au moyen par exemple d'une bande textile légère.

En référence aux figures 1A et 1B, la zone médiane 2a et les ailes d'accrochage 3a,3'a de l'orthèse 1a sont formées d'une pièce unique réalisée par exemple dans un premier silicone, de préférence un élastomère de silicone se présentant sous la forme d'une feuille d'environ 1 ou 2 mm d'épaisseur. Le premier silicone est par exemple du MED-4950 de la société NUSIL Silicone Technology. Ce premier silicone est choisi pour ses bonnes performances mécanique et notamment pour au moins l'une des caractéristiques suivantes :
- sa bonne résistance au déchirement,
- son allongement à la rupture élevé, supérieur à 600 % et de préférence supérieur à 670 %,
- sa force à 200 % d'allongement d'au moins 2 MPa et de préférence d'au moins 2.8 MPa,
- sa dureté entre 20 à 60 Shore A et de préférence entre 30 et 50 Shore A.

Ce premier silicone peut avantageusement être micro-perforé pour autoriser l'évacuation de la transpiration lors du port de l'orthèse 1a et en particulier en cas de pratique d'activité physique. L'orthèse 1a est ainsi apte à se déformer élastiquement et peut être tendue, appliquée sur une zone anatomique pour assurer son maintien. Le premier silicone est utilisé transparent ou teinté dans la masse par exemple au moyen d'une pigmentation biocompatible. On peut ainsi obtenir des orthèses 1a de couleurs variées, voire ayant des éléments de couleurs différentes.

Dans cet exemple, chaque pan de maintien supérieur et inférieur 4'a, 4a comporte une aile d'accrochage 3a, 3'a, deux zones de renfort 10a, 10'a, 11 a, 11'a et une zone médiane 2a.

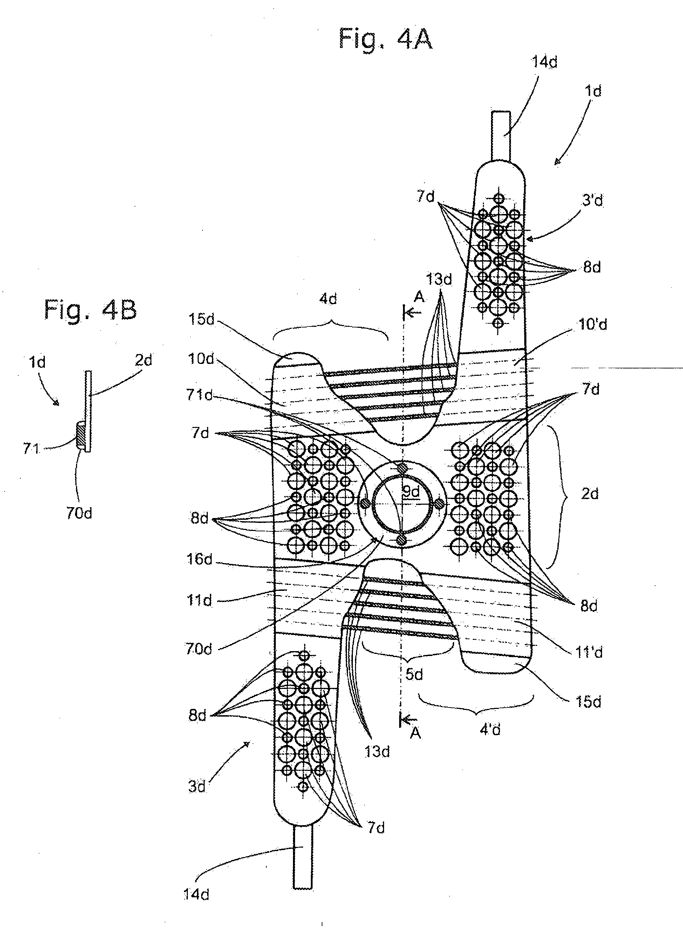

La zone médiane 2a de chaque pan de maintien supérieur et inférieur 4'a, 4a comporte une pluralité de premiers coussinets 7a distincts entre eux, répartis à intervalles réguliers sur des lignes prévues en quinconce les unes par rapport aux autres. Les premiers coussinets 7a sont réalisés dans un second silicone ayant des caractéristiques d'adhérence à la peau supérieures à celle du premier silicone. Cette adhérence est par exemple évaluée au moyen de l'un des tests d'adhésion connus tel que notamment le test JKR qui consiste à fabriquer une sphère (molle) à partir d'un matériau adhésif à tester puis à appuyer la sphère sur un substrat et à mesurer l'aire de contact de la sphère sur le substrat en fonction de la force F appliquée à la sphère, le test du Tack (probe-tack) qui consiste à mesurer les variations de déformation d'un adhésif placé entre deux pistons en fonction de la force devant être développée afin de les décoller, ou le test de pelage qui consiste à appliquer un morceau de ruban sur un panneau de test et à retirer le ruban sous un certain angle, appelé angle de pelage, la force mesurée une fois divisée par la largeur du ruban étant appelée force de pelage. Les premiers coussinets 7a définissent une première zone d'adhérence discontinue formant des moyens d'accrochage secondaires de l'orthèse 1a sur la zone anatomique. Le fait que cette première zone d'adhérence soit discontinue facilite la déformation de l'orthèse 1a pour suivre les mouvements de l'articulation sans que les premiers coussinets 7a ne soient décollés de la peau. Ainsi, la tenue de l'orthèse 1a en est améliorée. Le second silicone, dans lequel les premiers coussinets 7a sont formés, est de préférence un élastomère de silicone se présentant sous la forme d'un gel, par exemple du MED-4086 de la société NUSIL Silicone Technology. Les premiers coussinets 7a sont par exemple réalisés dans un silicone ayant une dureté inférieure à 1 Shore A et de préférence sensiblement égale à 55 type 000 Shore A (type « ultra soft elastomer »). Le second silicone est utilisé transparent ou teinté dans la masse. Dans cet exemple, les premiers coussinets 7a sont sensiblement circulaires et d'un diamètre d'environ 18 mm. Ils sont agencés selon quatre lignes espacées l'une de l'autre d'environ 10 mm. Dans une variante de réalisation non représentée, les premiers coussinets peuvent ne pas être alignés, voire être disposés de manière aléatoire. On peut également prévoir différentes zones dans lesquelles les premiers coussinets sont agencés selon des densités différentes. On peut également utiliser, sur une même orthèse, des premiers coussinets de formes et dimensions différentes (diamètre et/ou hauteur) adaptées aux spécificités de la zone anatomique au contact de laquelle ils sont destinés à être positionnés. On peut enfin prévoir des coussinets réalisés dans différents matériaux, certains coussinets définissant la première zone d'adhérence, les autres n'ayant pas les mêmes caractéristiques d'adhérence. Ainsi, comme décrit plus loin en référence aux figures 4A à 5B, certains coussinets peuvent comporter une substance active destinée à se diffuser vers la peau. Dans l'exemple illustré, chaque premier coussinet 7a est bombé et définit, par rapport au reste de la bande de tension, un premier relief autorisant la circulation de l'air autour de lui. La hauteur de chaque premier coussinet 7a est par exemple de 7 mm. Ainsi, les premiers coussinets 7a définissent ensemble, et avec la bande de tension, un réseau libre autorisant, en particulier lorsque l'orthèse 1a est portée, la circulation de l'air entre les premiers coussinets 7a. La zone anatomique est ainsi plus facilement aérée, limitant d'autant la sudation de la personne portant l'orthèse 1a. Les premiers coussinets 7a peuvent également avoir la forme de plots, cylindriques ou non. Les premiers coussinets 7a sont prévus pleins ou creux. Ils peuvent par exemple contenir une bulle réalisée dans un autre matériau, par exemple de l'air, un liquide ou tout autre matériau adapté. Outre les propriétés d'adhérence qu'il présente, le second silicone dans lequel les premiers coussinets 7a sont réalisés leur procure une bonne souplesse leur permettant, lorsqu'ils sont en contact avec la peau, de se déformer pour suivre l'anatomie. Ils améliorent ainsi le maintien de l'orthèse 1a.

La zone médiane 2a de chaque pan de maintien supérieur et inférieur 4'a, 4a comporte également des trous d'aération 8a (visibles sur la figure 1 B), traversant la bande de tension, disposés alignés et en quinconce de manière intercalée par rapport aux premiers coussinets 7a. Les trous d'aération 8a sont ainsi, par exemple, disposés à mi-distance des premiers coussinets 7a. Les trous d'aération 8a sont notamment destinés à autoriser le passage de l'air au travers de la bande de tension et coopèrent avec les premiers coussinets 7a pour que l'air puisse passer du réseau libre formé par ces premiers coussinets 7a vers l'extérieur de l'orthèse 1a et réciproquement. Ces trous d'aération 8a ont par exemple un diamètre d'environ 10 mm. Ils peuvent être de dimensions plus réduites, par exemple de l'ordre du micron pour autoriser une micro-aération. Ils peuvent également ne pas être circulaires. Les trous d'aération 8a participent à la limitation de la sudation et au confort pendant le port de l'orthèse 1a.

La zone de jonction prévue entre les pans de maintien supérieur et inférieur 4'a, 4a est pourvue d'un orifice 9a, traversant la bande de tension et apte à accueillir une partie de la zone anatomique, en l'occurrence la rotule du genou pour une genouillère. L'orifice 9a peut ainsi servir de repère pour le positionnement et l'accrochage de l'orthèse 1a qui peut ainsi être précis et efficace. Cet orifice 9a est par exemple sensiblement circulaire et d'un diamètre d'environ 6 cm. La périphérie de l'orifice 9a étant délimitée par la bande de tension réalisée dans le premier silicone, l'orifice 9a est apte à se déformer pour s'adapter aux différentes anatomies. L'orifice 9a est de plus entouré par un anneau de maintien 16a (visible sur la figure 1 B), concentrique et tangent à l'orifice 9a et formé par un anneau plein 70a et prévu du même côté de la bande de tension que les premiers coussinets 7a. L'anneau de maintien 16a a par exemple un diamètre intérieur d'environ 6 cm et une largeur d'environ 3 cm. Dans cet exemple, la bande de tension, dans sa zone médiane 2a, dépasse latéralement de l'anneau de maintien 16a. L'anneau de maintien 16a est avantageusement réalisé dans le même second silicone que les premiers coussinets 7a. Il définit ainsi un second coussinet (dans cet exemple annulaire) formant une seconde zone d'adhérence, coopérant avec la première zone d'adhérence des premiers coussinets 7a, pour favoriser la bonne tenue de l'orthèse 1a sur la peau. De plus, l'anneau de maintien 16a permet de renforcer la solidité de l'orthèse 1a autour de l'orifice 9a soumis à de grandes amplitudes d'étirements en flexion et en rotation. Dans cet exemple, l'anneau de maintien 16a est plein et simple.

Selon une variante de réalisation non représentée, l'anneau de maintien est creux et comporte un évidement annulaire non débouchant, ou une fente circulaire par exemple débouchant sur le dessus de l'anneau de maintien. Lorsque l'anneau de maintien comporte un évidement annulaire, ce dernier peut être réalisé dans un autre matériau, par exemple de l'air, un liquide ou tout autre matériau adapté. On peut également prévoir deux anneaux de maintien, ou plus, concentriques les uns par rapport aux autres, de diamètres différents et définissant entre eux des rainures.

Selon une autre variante de réalisation non représentée, l'orifice est remplacé par un simple évidement non débouchant formé dans l'épaisseur de la bande de tension et formant un logement apte à recevoir une partie de la rotule. Comme décrit plus loin, l'anneau de maintien peut par ailleurs comporter une substance active.

Les pans de maintien supérieur et inférieur 4'a, 4a comportent des zones de renfort 10a, 11a, 10'a, 11'a encadrant la zone médiane 2a, et donc décalées latéralement par rapport à cette zone médiane 2a. Ces zones de renfort 10a, 11a, 10'a, 11'a ont une rigidité supérieure à celle de la bande de tension dans sa zone médiane 2a et une dureté par exemple d'environ 85 Shore A et de préférence d'environ 70 à 80 Shore A. Les zones de renfort 10a, 11a, 10'a, 11'a peuvent être réalisées dans le premier silicone ou dans un silicone similaire. Dans cet exemple, les zones de renfort 10a, 11a, 10'a, 11'a forment une surépaisseur, orientée à l'opposé des premiers coussinets 7a par rapport à la bande de tension, par exemple d'environ 7 mm. Les zones de renfort 10a, 11a, 10'a, 11'a ont une largeur sensiblement constante et leurs bords intérieurs sont légèrement inclinés l'un vers l'autre par rapport à l'axe médian de l'orthèse 1a de sorte que la largeur de la zone médiane 2a du pan de maintien supérieur 4'a est supérieure à celle de la zone médiane 2a du pan de maintien inférieur 4a. Chaque zone de renfort 10a, 11a, 10'a, 11'a comporte des fourreaux 12a (visibles sur la figure 1A), sensiblement parallèles entre eux et par rapport au bord intérieur des pans de maintien supérieur et inférieur 4'a, 4a. Ces fourreaux 12a se présentent par exemple sous la forme d'alésages cylindriques d'environ 2 mm de diamètre et de préférence de 3 à 4 mm de diamètre, s'étendant sur une partie de la hauteur de chaque pan de maintien supérieur et inférieur 4'a, 4a, dont les extrémités se faisant face sont débouchantes et les extrémités opposées sont obturées. Les fourreaux 12a sont aptes à recevoir des éléments raidisseur 13a, par exemple des tiges cylindriques métalliques au nombre de cinq, d'environ 2 à 3 mm de diamètre, pouvant être réalisées en matériau à mémoire de forme tel que le Nitinol (nickel/titane, ou cuivre/aluminium/béryllium). Les éléments raidisseur 13a peuvent également être réalisés en polymère ou en autre matériau, par exemple en corde de silicone. De plus, les éléments raidisseur 13a ont une longueur inférieure à la distance séparant les extrémités opposées des fourreaux 12a. Les éléments raidisseur 13a sont ainsi bloqués par, et entre, les extrémités opposées et fermées des fourreaux 12a, extrémités entre lesquelles ils peuvent coulisser. Ils peuvent ainsi suivre les déformations de l'orthèse 1a lorsque celles-ci provoquent l'éloignement, le rapprochement ou la torsion des zones de renfort 10a, 11a, 10'a, 11'a entre-elles. Les éléments raidisseur 13a assurent ainsi une flexion contrôlée, une extension progressive, un maintien latéral, un contrôle, et un rappel en position de départ de l'orthèse 1a et de l'articulation. La rigidité des éléments raidisseur 13a entre les zones de renfort 10a, 11a, 10'a, 11'a d'un même côté droit ou gauche de l'orthèse 1a peut être modulée en faisant varier le nombre d'éléments raidisseur 13a utilisés et/ou les caractéristiques élastiques de ces éléments raidisseur 13a. Les raidisseurs connus permettent d'assurer une stabilité de l'orthèse selon l'axe de l'articulation. L'orthèse et les raidisseurs selon l'invention permettent de plus le contrôle progressif des mouvements.

Chaque pan de maintien supérieur et inférieur 4'a, 4a est prolongé, au-delà des zones de renfort supérieure gauche 10'a et inférieure droite 11a, par une aile d'accrochage 3'a, 3a, respectivement gauche et droite. Les ailes d'accrochage 3'a, 3a, sont par exemple formées dans le premier silicone et peuvent faire partie intégrante de la bande de tension, comme dans l'exemple illustré. Les ailes d'accrochage 3'a, 3a, comportent des trous d'aération 8a similaires à ceux de la zone médiane 2a. Ils peuvent bien entendu être différents dans leurs dimensions et/ou leur répartition. Chaque aile d'accrochage 3'a, 3a est pourvue, en son extrémité libre, d'une zone d'accrochage mâle ou femelle 14a apte à coopérer avec une zone d'accrochage femelle ou mâle complémentaire (non représentée) et prévue par exemple au dos de l'orthèse 1a. Les zones d'accrochage mâle ou femelle 14a sont par exemple de type Velcro. Selon une variante de réalisation l'orthèse comporte une boucle, par exemple en silicone rigide, couplée à une aile d'accrochage, l'autre aile d'accrochage étant formée par une bande de silicone pourvue d'ailettes déformables, cette bande étant apte à être passée dans la boucle et à être bloquée dans la boucle par ses ailettes lorsqu'une tension est appliquée sur l'aile d'accrochage correspondante. Une telle orthèse qui comporte par ailleurs des éléments raidisseur en silicone présente l'avantage majeur de pouvoir être entièrement réalisée en silicone.

La bande de tension est également prolongée, au-delà des zones de renfort supérieure droite 11'a, et inférieure gauche 10a, par des volets 15'a, 15a respectivement droit et gauche, de faible largeur. Les ailes d'accrochage 3'a, 3a, les zones d'accrochage mâles ou femelles 14a et les zones d'accrochage femelles ou mâles correspondantes 14a définissent les moyens principaux d'accrochage.

Ainsi, les moyens principaux d'accrochage sont distincts des moyens secondaires d'accrochage, ces derniers étant disposés entre les moyens principaux d'accrochage. La tenue de l'orthèse 1a sur la peau en est ainsi favorisée. En effet, outre la souplesse de la bande de tension qui permet d'encaisser les déformations de la peau, le fait que les premiers coussinets 7a et l'anneau plein 70a soient distincts les uns des autres, et dissociés des moyens principaux d'accrochage, permet d'optimiser l'adhérence des différentes zones d'adhérence en fonction des sollicitations prévues pour chacune de ces différentes zones d'adhérence.

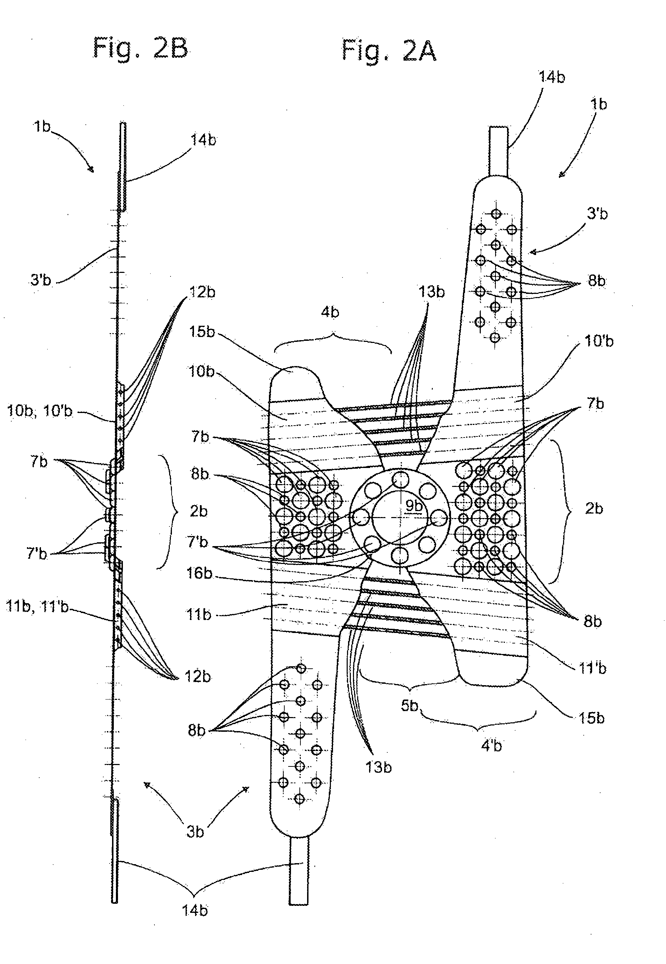

En référence aux figures 2A et 2B, l'orthèse 1b, selon le second mode de réalisation de l'invention, est sensiblement similaire à la précédente. Les éléments similaires portent le même numéro avec un indice b. Dans cet exemple, les premiers coussinets 2b sont agencés selon quatre lignes. L'orthèse 1b se distingue de la précédente, principalement par le fait que la bande de tension est formée de deux pans de maintien 4b, 4'b distincts et reliés par un anneau de maintien 16b entourant l'orifice. De plus dans cet exemple, l'anneau de maintien 16b comporte un anneau plein 70b, par exemple réalisé dans le premier silicone, cet anneau plein 70b portant des seconds coussinets 7'b réalisés dans le second silicone et sensiblement similaires aux premiers coussinets 7b. Les seconds coussinets 7'b peuvent néanmoins avoir une hauteur différente de celle des premiers coussinets 7b, par exemple supérieure. La seconde zone d'adhérence définie par l'anneau de maintien 16b est donc ainsi formée par une pluralité de seconds coussinets 7'b disposés autour de l'orifice 9b et donc discontinue. De plus, ces seconds coussinets 7'b autorisent le passage de l'air vers la rotule. Dans cet exemple, la bande de tension ne dépasse pas latéralement dans la zone médiane 2b de l'anneau de maintien 16b, et elle est discontinue au niveau de cet anneau de maintien 16b.

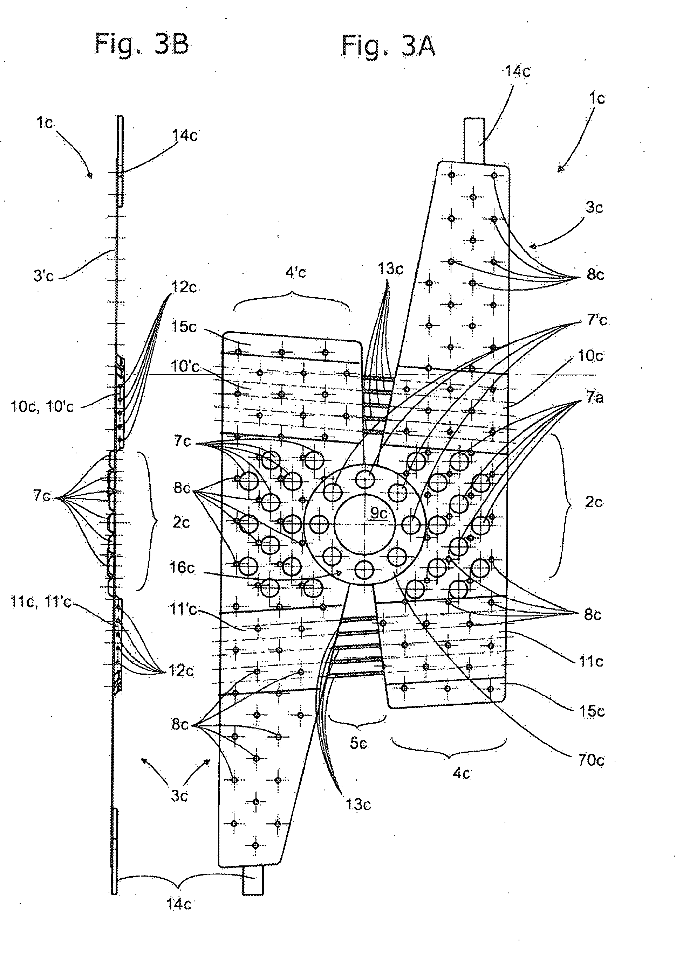

En référence aux figures 3A et 3B, l'orthèse 1c selon le troisième mode de réalisation de l'invention est sensiblement similaire à la précédente. Les éléments similaires portent le même numéro avec un indice c. Elle s'en différencie principalement par la forme des pans de maintien 4c, 4'c qui est plus angulaire, et par les premiers coussinets 7c qui sont répartis de manière plus étalée. De plus, les trous d'aération 8c de la zone médiane 2c ne sont pas prévus à mi-distance entre deux premiers coussinets 8c. Par ailleurs l'intégralité de la surface des pans de maintien 4c, 4'c, des volets 15'c, 15c et des ailes d'accrochage 3c, 3'c est pourvue de trous d'aération 8c. Cette orthèse 1c se différencie également de la précédente par le fait que l'aile d'accrochage supérieure 3'c est une aile gauche et non droite et que l'aile d'accrochage inférieure 3c est une aile droite et non gauche.

En référence aux figures 4A et 4B, l'orthèse 1d selon le quatrième mode de réalisation de l'invention est sensiblement similaire à l'orthèse 1a des figures 1A et 1B. Les éléments similaires portent le même numéro avec un indice d. Elle s'en différencie principalement par le fait que l'anneau de maintien 16d comporte un anneau plein 70d, par exemple réalisé dans le premier silicone, cet anneau plein 70d étant pourvu de quatre logements sensiblement circulaires, répartis uniformément le long de l'anneau plein 70d. Chaque logement comporte un élément actif 71d se présentant sous la forme d'un plot sensiblement circulaire (représenté par une forme noircie). Chaque élément actif 71d est par exemple réalisé dans le second silicone et comporte une substance active par exemple antidouleurs et/ou anti-inflammatoire et/ou huiles essentielles, telle que par exemple des oestrogènes, de la nicotine, de la Lidocaïne, cette substance active étant apte à se diffuser au travers de l'élément actif 71d vers la peau pour assurer l'administration par voie transcutanée de la substance active. La substance active peut être dissoute ou dispersée (type matriciel ou type réservoir) dans la masse de l'élément actif 71d. L'augmentation de la température des éléments actifs 71d en contact avec la peau, augmentation renforcée par la contention et les frottements de la peau contre des éléments actifs 71d, favorise la diffusion de la substance active. Les éléments actifs peuvent bien entendu être de nature et/ou de forme différentes. Au terme de la diffusion de la substance active, l'élément actif 71d peut être extrait de son logement et changé. De plus, dans ce quatrième mode de réalisation, les ailes d'accrochage 3'd, 3d, sont également pourvues de premiers coussinets 7d favorisant la bonne tenue de l'orthèse 1d sur la zone anatomique.

Selon une variante de réalisation non représentée, les éléments actifs peuvent se présenter sous la forme de coussinets ayant au moins une face adhésive apte à adhérer sur la face interne de l'orthèse, déplaçables et interchangeable. Ils participent alors à la circulation de l'air entre la zone anatomique et l'orthèse.

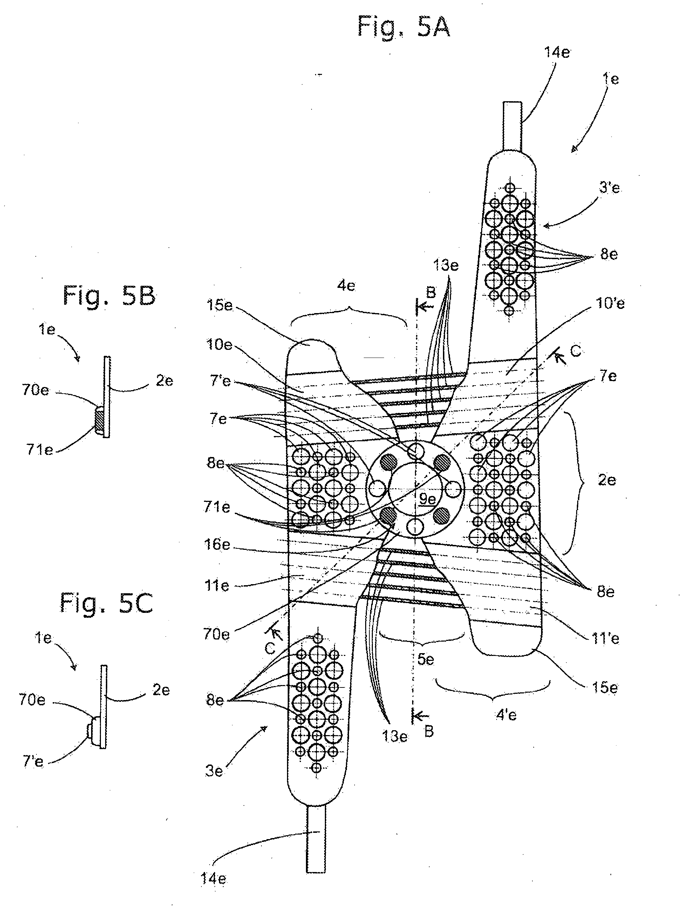

En référence aux figures 5A, 5B et 5C, l'orthèse 1e selon le cinquième mode de réalisation de l'invention est sensiblement similaire à une combinaison des orthèses 1a et 1d des figures 1A, 1 B, 4A, 4B. Les éléments similaires portent le même numéro avec un indice e. L'orthèse 1e se différencie des orthèses 1a et 1d, principalement par le fait que l'anneau de maintien 16e comporte un anneau plein 70e, par exemple réalisé dans le premier silicone, cet anneau plein 70e étant pourvu simultanément de seconds coussinets 7'e et de logements comportant un élément actif 71e.

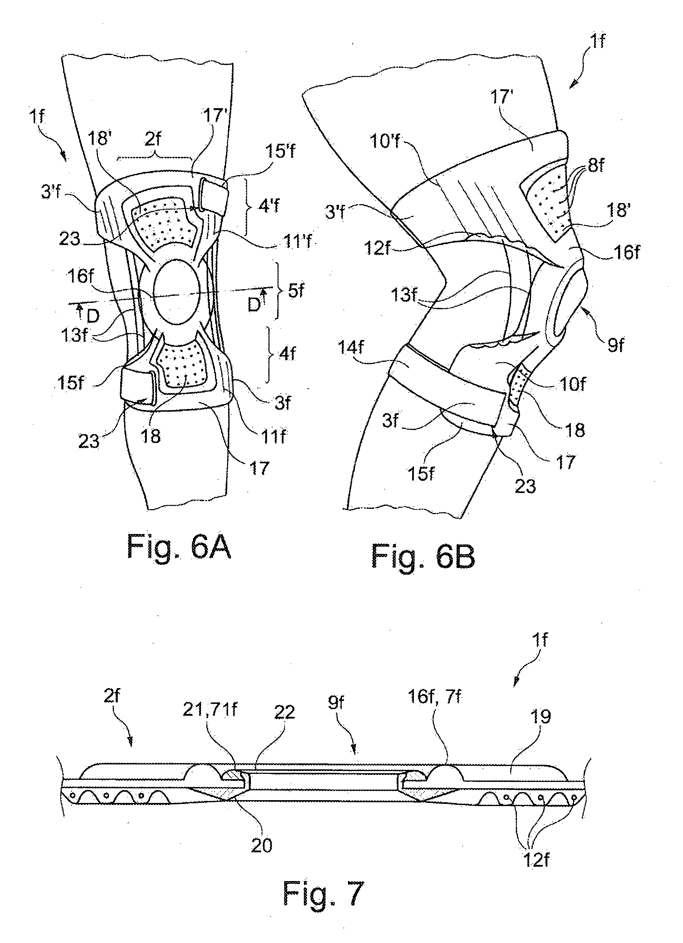

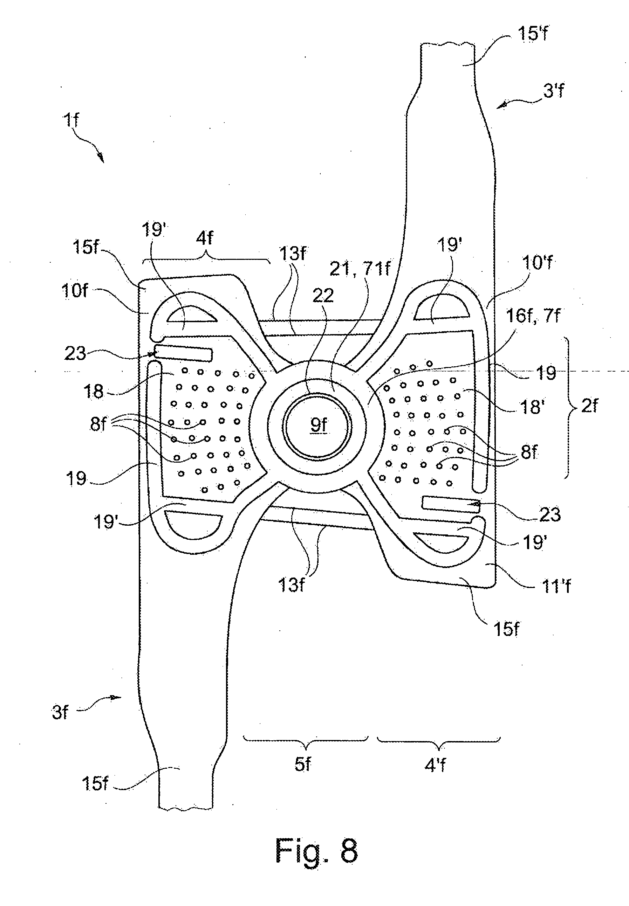

En référence aux figures 5A, 5B, 7 et 8, l'orthèse 1f selon le sixième mode de réalisation de l'invention, est sensiblement similaire aux orthèses des figures 1A et 5A. Les éléments similaires portent le même numéro avec un indice f. Selon ce mode de réalisation, la bande de tension est formée de deux pans de maintien 4f, 4'f distincts et reliés par un anneau de maintien 16f entourant l'orifice 9f. L'anneau de maintien 16f, réalisé dans le second silicone, forme en partie les moyens secondaires d'accrochage. Les pans de maintien supérieur et inférieur 4'f, 4f de l'orthèse 1f sont chacun formés respectivement par une armature 17, 17' entourant un évidement couvert par un voile 18, 18'. Le voile 18, 18' est de préférence traversé par des trous d'aération 8f (visibles sur les figures 6A, 6B) facilitant le passage de l'air au travers de l'orthèse 1f et limitant les effets négatifs de la sudation. L'armature 17, 17' et le voile 18, 18' peuvent être réalisés dans le même premier silicone. L'armature 17, 17' définit une forme en huit, visible sur les figures 6A et 6B, délimitant deux zones fermées, une zone par pan de maintien 4f, 4'f. Ces zones fermées destinées à entourer des zones anatomiques, sont reliées entre elles par l'anneau de maintien 16f, concentrique et tangent à l'orifice 9f. Cet anneau de maintien 16f définit par rapport à la zone active 2f un premier relief. L'orifice 9f présente des dimensions inférieures aux dimensions intérieures de l'anneau de maintien 6f, définissant ainsi une lèvre d'appui 20 (visible sur la figure 7) entourée par l'anneau de maintien 16f. Cette lèvre d'appui 20 sert de support à un anneau amovible 21 définissant, par rapport à la zone active 2f, un premier relief. Cet anneau amovible 21 comporte une substance active, telle que décrite précédemment, destinée à se diffuser vers la peau pour traiter la zone anatomique. La lèvre d'appui 20 est prolongée par un retour 22 orienté vers le coté de l'orthèse 1f pourvu de l'anneau de maintien 16f. L'anneau de maintien 16f, le retour 22 et la lèvre d'appui 20 définissent une gorge dans laquelle l'anneau amovible 21 contenant le principe actif est placé. Lorsque l'orthèse 1f est portée, l'anneau amovible 21 peut être inséré ou retiré de la gorge en étant déformé élastiquement et passé au travers de l'orifice 9f. Lorsque l'orthèse 1f n'est pas portée, l'anneau amovible 21 peut être mis en place par l'arrière de l'orthèse 1f. L'anneau amovible 21 peut ainsi être facilement mis en place, et remplacé après diffusion complète de la substance active qu'il contient. L'augmentation de la température provoquée par la contention lors du port de l'orthèse 1f favorise la diffusion des principes actifs contenus dans l'anneau amovible 21. L'orthèse 1f comporte de plus un bourrelet 19 en relief, visible sur les figures 7 et 8, prévu du même coté de l'orthèse 1f que l'anneau de maintien 16f. Ce bourrelet 19 est ainsi apte à être plaqué contre la zone anatomique lorsque l'orthèse 1f est portée. Le bourrelet 19 est avantageusement réalisé dans le second silicone permettant de favoriser l'adhésion de l'orthèse 1f contre la peau, et en particulier autour de la zone anatomique à traiter. Il forme ainsi en partie les seconds moyens d'accrochage. Le bourrelet 19 définit une forme en huit sensiblement similaire à la forme en huit définit par l'armature 17, 17'. Cette double forme en huit contribue à la solidité, à l'efficacité et au confort de l'orthèse 1f. La forme en huit délimitée par le bourrelet 19 est discontinue et interrompue au niveau de fentes 23 traversantes prévues dans les volets droit 15'f et gauche 15f prolongeant les pans de maintien supérieur et inférieur 4'f, 4f. Ces fentes 23 sont aptes à recevoir le passage des bandes d'accrochage 3'f, 3f afin qu'elles forment chacune une boucle. L'extrémité de la bande d'accrochage 3'f, 3f est par exemple pourvue de zones de type velcro aptes à coopérer entre elles, une fois rabattues l'une sur l'autre, comme illustré sur les figure 6A et 6B. Le bourrelet 19 comporte en outre des longerons de renfort 19' prévus dans les arrondis de la forme en huit pour servir d'appui et de zone d'adhérence. Dans ce mode de réalisation, l'ensemble de l'orthèse 1f, exceptée les zones de type velcro, est réalisé en silicones. De manière avantageuse, les zones de type velcro peuvent être remplacées par des zones mâles et femelles réalisées elles aussi en silicone, ces aptes zones mâles et femelles étant aptes à coopérer pour s'accrocher entre elles, par exemple des picots et des orifices. La figure 6B permet de visualiser la déformation élastique des raidisseurs 13f lors de la flexion du genou, dans cet exemple au nombre de deux, dont les extrémités sont logées dans les fourreaux 12f.

Selon une variante de réalisation non représentée, la zone médiane et les zones de renfort et/ou les ailes d'accrochage, sont formées par des éléments distincts associés entre eux.

Selon une autre variante de réalisation non représentée, la bande de tension n'est pas prévue pour faire le tour du membre ou du torse mais pour être accrochée directement sur la peau au moyen par exemple d'une zone d'adhérence principale périphérique. Elle peut également être destinée à être fixée par tout autre moyen adapté.

Selon encore une autre variante de réalisation non représentée, l'orthèse comporte des surfaces d'adhérences intégrées à la bande de tension et non proéminentes par rapport à la même bande de tension. Il peut s'agir de plots de faible épaisseur, par exemple circulaires, reçus dans des logements prévus dans l'épaisseur de la bande de tension. Dans cette configuration, les plots ne font pas office de coussinets amortissant, leur fonction se limitant à assurer l'adhérence de la bande de tension sur la peau. Il n'y a donc pas de réseau libre entre les plots, l'aération étant assurée par la perforation (voir micro perforation) de la bande de tension.

Selon une autre variante de réalisation non représentée, certains premiers coussinets, voire l'anneau de maintien, sont prévus déplaçables. Dans ce cas, ils ne sont pas solidaires de la bande de tension par rapport à laquelle, une fois positionnés sur la face interne de l'orthèse, ils restent en place du fait de leurs propriétés d'adhérence à cette face interne.

Selon encore une autre variante de réalisation non représentée, de éléments actifs peuvent être prévus entre les premiers coussinets portés par les pans de maintien.

Selon encore une autre variante de réalisation non représentée, les premiers coussinets combinent les propriétés d'adhérence et celles des éléments actifs.

Dans les exemples décrits, la zone active comporte également les zones de renfort et les ailes d'accrochage également en silicone, elle s'étend donc au-delà de la zone médiane. Il peut bien entendu en être autrement, notamment lorsque la zone médiane est prolongée latéralement par des ailes d'accrochage non élastiquement déformable.

Pour mettre en place l'orthèse 1a-1f selon l'invention et plus particulièrement la genouillère, on positionne la bande de tension en regard du genou. Lorsque l'orthèse 1*f* comporte un anneau amovible 21, on place éventuellement ce dernier dans la gorge qui lui est destiné, avant la pose de l'orthèse 1f. Une fois l'orthèse 1a-1f placée en regard du genou, les pans de maintien 4'a-4'f, 4a-4f sont alors respectivement au-dessus et en-dessous du genou et les ailes d'accrochage 3'a-3'f, 3a-3f ouvertes de part et d'autre du genou. On tend latéralement la bande de tension puis on applique l'orthèse 1a-1f de manière à ce que la rotule se loge dans l'orifice 9a-9f définissant un évidement rotulien et que les ailes d'accrochage 3'a-3'f, 3a-3f s'étendent latéralement, sensiblement horizontalement de part et d'autres de la rotule. Le second silicone formant l'anneau de maintien 16a-16f, et éventuellement le bourrelet 19, adhèrent instantanément autour de la rotule et de manière plus large. De plus, la malléabilité du silicone permet à l'orthèse 1a-1f de se conformer à la géométrie de la rotule, en entrant notamment facilement dans les cavités de formes très variées. On maintient la bande de tension tendue et on plaque l'orthèse 1a-1f sur la peau afin que les premières zones d'adhérence assurent une adhésion sensiblement uniforme sur toutes leurs surfaces. Le second silicone des premiers coussinets 7a- 7f, éventuellement de l'anneau de maintien 16a-16fet du bourrelet 19, étant de faible dureté, au contact de la peau il assure un rembourrage ainsi qu'une adhérence instantanée. Tout en maintenant la bande de tension sous tension et les premiers coussinets 7a- 7f, anneau de maintien 16a-16f et bourrelet 19, appliqués sur la peau, on rabat l'aile d'accrochage supérieure 3'a-3'f vers l'arrière du genou pour ceinturer la face postérieure de la cuisse jusqu'à ce que la zone d'accrochage 14a- 14f de l'aile d'accrochage supérieure 3'a-3'f soit en regard de la zone d'accrochage complémentaire (non représentée) prévue sur le devant de l'orthèse 1a-f. On règle alors la tension appliquée sur la bande de tension en fonction de la contention désirée. Pendant cette étape de réglage, les premiers coussinets 7a- 7f, anneau de maintien 16a-16f et bourrelet 19, restent en contact adhésif avec la peau. On réalise la même opération de ceinturage avec l'aile d'accrochage inférieure 3a-3f que l'on rabat, en sens opposée à l'aile d'accrochage supérieure 3'a-3'f vers l'arrière du genou. Les ailes d'accrochage supérieures 3'a-3'f et inférieures 3a-3f permettent ainsi d'assurer une tension progressive autour du genou. Comme elles sont prévues sensiblement symétriques entre-elles par rapport à l'axe médian de l'orthèse 1a-1f, elles permettent d'équilibrer les efforts appliqués à la bande de tension de la genouillère et donc les efforts appliqués par la genouillère au genou. La bande de tension étant élastique, les efforts de contention sont appliqués aussi bien sur la partie antérieure que sur la partie postérieure du genou. Le premier silicone micro aéré, dont l'allongement à la rupture est important, fournit l'élasticité nécessaire au réglage de la contention, en assurant une tension progressive, en fonction du périmètre de l'articulation, ainsi que la perméabilité nécessaire au confort de l'orthèse 1a-1f. La combinaison des premiers et second silicones permet ainsi d'associer élasticité et déformation pour obtenir une contention efficace tout en autorisant la mobilisation temporaire de l'articulation. La mise en place de l'orthèse 1a-1f selon l'invention est facilitée par le fait que la bande de tension est ouverte. Elle peut ainsi être enfilée facilement et ajustée sans création de surépaisseur. Les premiers et seconds coussinets 7a-7f, 7'a-7'f assurent un maintien provisoire le temps que les ailes d'accrochage 3'a-3'f, 3a-3f fassent le tour de la zone anatomique. L'élasticité de la bande de tension couplée à l'adhérence des premiers et seconds coussinets 7a-7f, 7'a-7'f, anneau de maintien 16a-16f et bourrelet 19, permet d'utiliser une même orthèse 1a-1f pour des morphologies variées. Ainsi, le nombre de tailles nécessaires pour couvrir l'ensemble des morphologies est plus limité qu'avec les orthèses actuelles. La mise en place d'une orthèse de cheville, de poignet, d'épaule ou de coude selon l'invention s'effectue de manière similaire à celle de la genouillère.

Pendant le port de l'orthèse 1a-1f, l'effet massant produit par les propriétés, du second silicone des premiers coussinets 7a- 7f de l'anneau de maintien 16a-16f et du_bourrelet 19, augmente-le maintien et le confort. De plus, le réseau libre d'aération formé par les premiers reliefs des premiers coussinets 7a- 7f permet d'assurer une aération et une ventilation augmentée par la micro perforation du premier silicone. L'orthèse 1a-1f selon l'invention convient donc à toute personne pratiquant une activité physique. L'adhérence des premiers coussinets 7a- 7f, de l'anneau de maintien 16a-16f et du bourrelet 19, permet de lutter efficacement contre l'effet glissement de la bande de tension. L'orthèse 1a-1f n'a pas besoin d'être serrée de manière excessive pour tenir en place. Les ailes d'accrochage 3'a-3'f, 3a-3f laissent libre le creux poplité à l'arrière du genou, diminuant ainsi l'effet garrot et/ou de cisaillement généré par la plupart des orthèses connues lors de la flexion de la jambe. Le nombre d'éléments raidisseur 13a-13f est de préférence ajusté avant la mise en place de l'orthèse 1a-1f afin de renforcer ou de réduire l'effort de flexion ou de rotation à fournir pour mobiliser le genou. La position des éléments raidisseur 13a-13f peut également être ajustée en utilisant différents fourreaux 12a-12f. Ce réglage permet notamment de placer les éléments raidisseur 13a-13f en fonction de la morphologie du porteur, notamment pour respecter au maximum les axes de l'articulation. Les mouvements de rotation interne, externe, la flexion ou l'extension de l'articulation ne sont pas figés mais accompagnés. Les zones de renfort 10a-10f, 10'a-10'f soutiennent et limitent les tensions des ligaments. Grâce aux propriétés hydrophobes du silicone, l'orthèse 1a-1f peut être portée en milieu humide, voire immergée, sans altération de son efficacité par exemple pour la pratique des sports nautiques, de la plongée, ... L'orthèse 1a-1f peut par ailleurs être rincée à l'eau chaude, voire lavée sans altération de ses propriétés. En effet, l'utilisation de silicones permet d'obtenir des orthèses réutilisables, lavables, supportant sans problème les variations de température et présentant, grâce à leur très bonne résistance à l'oxygène et à l'ozone, une bonne stabilité au vieillissement. Les silicones ont également une bonne résistance aux méthodes de stérilisation telle que stérilisation à l'alcool, aux rayons gamma, avec faisceau d'électrons, ... Les orthèses 1a-1f selon l'invention peuvent donc être stérilisées. Les silicones sont biocompatibles avec les tissus humains et les fluides corporels et n'irritent ni la peau ni les autres organes. Les orthèses en silicones peuvent donc être portées, pendant de longues périodes, sans risque de trouble cutané. On utilisera néanmoins de préférence un silicone de grade médical, inerte biologiquement, qui permet de garantir que le port prolongé de l'orthèse 1a-1f ne risque pas d'engendrer le développement de bactéries. Les silicones utilisés permettent notamment de répondre aux normes FDA21 CFR 177-2600, USPCLASS VI et ISO 10993. Afin de garantir ces propriétés à l'orthèse 1a-1f, on s'assurera que la zone médiane 2a-2f de la bande de tension soit exempte de matériau textile. Un autre avantage de l'orthèse 1a-1f selon l'invention réside dans le fait que le silicone ne colore ni ne corrode pas les matériaux avec lesquels il est en contact. Cette orthèse 1a-1f peut donc être portée en combinaison avec tout type de vêtements. Lorsqu'elle est fabriquée en silicone transparent, l'orthèse 1a-1f peut être portée de manière discrète. Cette configuration permet de plus de voir l'articulation au travers de l'orthèse 1a-1f.

Pour retirer l'orthèse 1a-1f, on détache la zone d'accrochage 14a-14f de l'aile d'accrochage inférieure 3a-3f et on ramène l'aile d'accrochage inférieure 3a-3f vers le devant du genou tout en réduisant progressivement la tension appliquée sur la bande de tension. On procède de même avec l'aile d'accrochage supérieure 3'a-3'f. Ensuite, on écarte la bande de tension afin de décoller les premiers coussinets 7a- 7f et éventuellement second coussinets 7'b, 7'f. L'adhérence des premiers et seconds coussinets 7a-7f, 7'b, 7'f disparaît sans contrainte et de manière indolore pour le porteur de l'orthèse 1a-1f.

Entre deux utilisations de l'orthèse 1a-1f, les premiers et seconds coussinets 7a- 7f, 7'b, 7'f conservent leur pouvoir adhérent si bien qu'aucun dépôt d'un produit adhérent par pulvérisation ou toute autre technique n'est nécessaire avant sa réutilisation. Pour rafraichir le pouvoir adhérent des premiers et seconds coussinets 7a-7f, 7'b, 7'f, l'orthèse 1a-1f peut être lavée en machine, à l'éponge ou au moyen d'une brosse douce.

Les technologies de transformation et de mise en oeuvre des silicones sont multiples. Elles seront choisies en fonction des caractéristiques produits recherchées, de l'intérêt économique des produits fabriqués et des investissements à réaliser pour les outils de production. On pourra notamment utiliser les techniques de calandrage, moulage, surmoulage, compression, injection-moulage, injection-compression, extrusion, vulcanisation à froid, collage, ... Les propriétés du silicone permettent d'obtenir des duretés, allongements et indices d'adhérence ou propriétés mécaniques en fonction du résultat recherché. On décrit ci-après différents modes de réalisation des orthèses précédemment décrites.

Selon un premier mode de réalisation de l'orthèse 1a-1f selon l'invention, le contour externe des différents éléments (bande de tension, pans de maintien, zones de renfort...) sont découpés dans une feuille de silicone disposée dans une calandre au moyen d'outils délimitant le contour de ces éléments. Les premiers et seconds coussinets 7a- 7f, 7'b, 7'f, anneau de maintien 16a-16f et bourrelet 19, sont avantageusement obtenus par moulage. Les différents éléments sont ensuite positionnés les uns par rapport aux autres puis assemblés par vulcanisation au moyen d'un produit de vulcanisation ou par collage. On utilise de préférence une vulcanisation à froid qui permet de renforcer la cohésion de des molécules de chaque élément.

Selon un second mode de réalisation de l'orthèse 1a-1f selon l'invention, les différents éléments (bande de tension, pans de maintien, ...) sont obtenus par moulage de compression de matière première de haute consistance, ou par injection de silicone liquide basse consistance. La technique de l'injection qui permet de limiter les investissements nécessaires en termes d'outillage industriel sera préférée. L'outillage consiste notamment, pour chaque élément, en deux demi-coquilles formant le moule, chaque demi-coquille comportant, en négatif, une partie de l'élément à mouler. Le remplissage du moule peut être assuré manuellement, de manière semi-automatique ou automatique. Après remplissage du moule, la cuisson permet d'apporter l'énergie nécessaire à une cohésion durable de chaque élément. Cette étape de cuisson peut être suivie par une étape de finition au cours de laquelle on ébavure les bords de l'élément. L'orifice 9a-9f est obtenu directement par moulage ou lors d'une étape de découpage ultérieure. Les trous d'aération 8a-8f peuvent être formés lors de cette opération de moulage ou lors d'une opération de découpage ultérieure. Les premiers et seconds coussinets 7a- 7f, 7'b, 7'f, peuvent être surmoulés directement sur la bande de tension ou moulés séparément puis assemblés sur la bande de tension par vulcanisation au moyen d'un produit de vulcanisation ou par collage. De même, l'anneau de maintien 16a-16f et le bourrelet 19 peuvent être surmoulés directement ou moulés séparément puis collés à la bande de tension. Les zones de renfort 10a-10f, 11a-11f, 10'a-10'f, 11'a-11'f peuvent être surmoulées sur la bande de tension. Elles peuvent également être obtenues par extrusion. Les premiers et seconds coussinets 7a- 7f, 7'b, 7'c, 7'f et l'anneau de maintien 16a-16f, en second silicone peuvent également être moulés en même temps que la bande de tension en premier silicone.

Les différentes étapes des modes de réalisation décrits précédemment peuvent bien entendu être combinées.

Pour augmenter davantage la stabilité de l'orthèse 1a-1f, il est possible de recouvrir tout ou partie de la surface interne de l'orthèse 1a-1f, en particulier la surface interne des zones de renfort 10a-10f, 11a-11f, 10'a-10'f, 11'a-11'f, au moyen d'un silicone par exemple de type MED 4086 formant une « peau autocollante » améliorant l'adhérence de la face interne de l'orthèse 1a-1f sur la peau. Cette « peau autocollante » peut être appliquée par enduction, vaporisation spatulage ou toute autre technique adaptée.

### Possibilités d'application industrielle

La présente invention concerne le domaine des matériels orthopédiques et notamment les orthèses du genou, de la cheville, du poignet et de l'épaule, utilisées lors de la mobilisation ou de l'immobilisation temporaire de l'articulation. Elle s'applique également à tous types de maintien nécessitant adhérence et élasticité du dispositif sur un membre ou sur le tronc, par exemple pour des ceintures lombaires.

La présente invention concerne également tout autre type de complexe de traitement tel que notamment les pansements et timbres transdermiques.

Il est bien entendu que les exemples décrits ne sont que des illustrations particulières et en aucun cas limitatives des domaines d'application de l'invention. L'Homme de l'art pourra apporter des aménagements de taille, de forme et de matériau à l'exemple de réalisation particulier et du procédé d'installation sans pour autant sortir du cadre de la présente invention.

## Revendications

1. Complexe de traitement (1a-1f) pour zone anatomique du corps humain, ledit complexe de traitement (1a-1f) comportant au moins une zone active (2a-2f) pour traiter ladite zone anatomique, ladite zone active (2a-2f) étant couplée à des moyens principaux d'accrochage (3a-3f,3'a-3'f,14a-14f) prévus de part et d'autre de ladite zone active (2a-2f) et aptes à solidariser ledit complexe de traitement (1a-1f) à ladite zone anatomique, les bords latéraux dudit complexe de traitement (1a-1f) étant disjoints et non reliés entre eux au moins lorsque ledit complexe de traitement (1a-1f) n'est pas porté, **caractérisé en ce que** ladite zone active (2a-2f) est au moins en partie réalisée dans un premier silicone, **en ce que** ledit complexe de traitement (1a-1f) comporte des moyens secondaires d'accrochage (7a-7f,7'b,7'c,7'f,16a-16f,19), distincts desdits moyens principaux d'accrochage (3a-3f,3'a-3'f,14a-14f), disposés entre lesdits moyens principaux d'accrochage (3a-3f,3'a-3'f ,14a-14f) et couplés à ladite zone active (2a-2f), lesdits moyens secondaires d'accrochage (7a-7f,7'b,7'c,7'f,16a-16f,19) comportant au moins une première zone d'adhérence réalisée au moins en partie dans un second silicone ayant des caractéristiques d'adhésion à la peau supérieures à celles dudit premier silicone, ladite première zone d'adhérence étant apte à coopérer avec la peau de ladite zone anatomique pour au moins contribuer à l'adhésion de ladite zone active (2a-2f) sur ladite peau.

2. Complexe de traitement (1a-1f) selon la revendication précédente, **caractérisé en ce que** ladite première zone d'adhérence comporte au moins un coussinet (7a-7f,7'b,7'c,7'f,16a-16f,19) ayant une forme bombée, définissant par rapport à ladite zone active (2a-2f) un premier relief.

3. Complexe de traitement (1a-1f) selon la revendication précédente, **caractérisé en ce que** ladite première zone d'adhérence comporte une pluralité de coussinets (7a-7f,7'b,7'c,7'f) en silicone ayant chacun une forme bombée, définissant par rapport à ladite zone active (2a-2f) des premiers reliefs distants les uns des autres agencés pour ménager entre eux un réseau libre apte à autoriser la circulation de l'air entre ladite zone anatomique et ladite zone active (2a-2f) lorsque ledit complexe de traitement (1a-1f) est en appui contre ladite zone anatomique.

4. Complexe de traitement (1a-1f) selon la revendication 1, **caractérisé en ce que** ladite zone active est pourvue d'un orifice (9a-9f) apte à accueillir une partie de la zone anatomique, **en ce que** ladite première zone d'adhérence comporte un anneau de maintien (16a-16f) en silicone entourant ledit orifice (9a-9f), ledit anneau de maintien (16a-16f) définissant par rapport à ladite zone active (2a-2f) un premier relief.

5. Complexe de traitement (1f) selon la revendication 4, **caractérisé en ce que** ledit orifice (9f) présente des dimensions inférieures aux dimensions intérieures dudit anneau de maintien (16f) de manière à définir une lèvre d'appui (20) entourée dudit anneau de maintien (16f) et entourant ledit orifice (9f), ladite lèvre d'appui (20) étant apte à servir de support à un anneau amovible (21) logé dans ledit anneau de maintien (16f).

6. Complexe de traitement (1f) selon la revendication précédente, **caractérisé en ce que** ladite lèvre d'appui (20) est prolongée par un retour (22) orienté vers le coté de ladite orthèse (1f) comportant ledit anneau de maintien (16f), ledit retour (22) définissant, avec ladite lèvre d'appui (20) et ledit anneau de maintien (6f), une gorge apte à recevoir ledit anneau amovible (21) et à participer à son maintien.

7. Complexe de traitement (1a-1f) selon l'une quelconque des revendications précédentes, **caractérisé en ce qu'**il est pourvu d'au moins un élément actif (71d-71f) comportant au moins une substance active apte à se diffuser vers ladite peau pour assurer l'administration par voie transcutanée de ladite substance active.

8. Complexe de traitement (1f) selon les revendications 5 et 7, **caractérisé en ce qu'**il comporte au moins un anneau amovible (21) comportant au moins une substance active et formant ledit élément actif (71d-71f), ledit anneau amovible (21) étant inséré entre ladite lèvre d'appui (20) et ledit anneau de maintien (16f).

9. Complexe de traitement (1a-1f) selon l'une quelconque des revendications précédentes, **caractérisé en ce qu'**au moins ladite zone active (2a-2f) est exempte de matériau textile.

10. Orthèse (1a-1f) pour zone anatomique du corps humain, ladite orthèse (1a-1f) comportant au moins une bande de tension apte à être déformée élastiquement, tendue et appliquée sur ladite zone anatomique pour assurer son maintien, **caractérisée en ce qu'**elle se présente sous la forme d'un complexe de traitement (1a-1f) selon l'une quelconque des revendications précédentes, ladite orthèse (1a-1f) comportant une zone médiane (2a-2f) définissant au moins en partie ladite zone active et pourvue sur une de ses surfaces d'au moins une partie desdits moyens secondaires d'accrochage (7a-7f,7'b,7'c,7'f,16a-16f,19).

11. Orthèse (1a-1f) selon la revendication précédente, **caractérisée en ce qu'**elle est formée par au moins deux pans de maintien (4a-4f,4'a-4'f) juxtaposés et reliés entre eux, au niveau de ladite zone médiane (2a-2f), par une zone de jonction délimitant les bords latéraux de ladite zone médiane (2a-2f) de ladite orthèse (1a-1f), ladite zone de jonction ayant une largeur inférieure à celle desdits pans de maintien (4a-4f,4'a-4'f).

12. Orthèse (1f) selon la revendication 11, **caractérisée en ce qu'**au moins un desdits pans de maintien (4f, 4'f) comporte au moins une armature (17, 17'), au moins un évidement entouré par ladite armature (17, 17') et un voile (18, 18') en silicone recouvrant ledit évidement.

13. Orthèse (1f) selon la revendication 10, **caractérisée en ce qu'**au moins un desdits pans de maintien (4f, 4'f) est pourvu d'au moins un bourrelet (19) en relief apte à être plaqué contre ladite zone anatomique lorsque ladite orthèse (1f) est portée et formant ledit coussinet, ledit bourrelet (19) étant agencé pour délimiter une zone au moins en partie fermée apte à entourer au moins une partie de ladite zone anatomique.

14. Orthèse (1f) selon l'une des revendications 12 et 13, **caractérisée en ce que** l'un au moins desdits armature (17, 17'), bourrelet (19), définit une forme en huit délimitant au moins deux zones fermées destinées à être placées en regard de ladite zone anatomique et reliées auxdits moyens principaux d'accrochage.

15. Orthèse (1a-1f) selon la revendication 10, **caractérisée en ce qu'**elle comporte au moins deux paires de zones de renfort (10a-10f, 10'a-10'f), distantes et décalées latéralement par rapport au centre de ladite zone médiane (2a-2f), chaque zone de renfort (10a-10f, 10'a-10'f) ayant une rigidité supérieure à celle de ladite zone médiane (2a-2f), les zones de renfort (10a-10f, 10'a-10'f) d'une même paire étant disposées en regard l'une de l'autre et reliées entre-elles par au moins un élément raidisseur (13a-13f) élastiquement déformable et ayant une rigidité supérieure à celle de ladite zone médiane (2a-2f).

16. Orthèse (1a-1f) selon la revendication précédente, **caractérisée en ce que** chaque zone de renfort (10a-10f, 10'a-10'f) comporte au moins un fourreau (12a-12f) apte à recevoir une extrémité dudit élément raidisseur (13a-13f), lesdits fourreaux (12a-12f) des zones de renfort (10a-10f, 10'a-10'f) constituant une paire, étant prévus en regard l'un de l'autre, **en ce que** ledit élément raidisseur (13a-13f) a une longueur inférieure à la distance séparant les extrémités opposées desdits fourreaux (12a-12f) l'autorisant à coulisser entre lesdites extrémités pour suivre la déformation angulaire de ladite orthèse (1a-1f), sans dépasser lesdits fourreaux (12a-12f).

## Patentansprüche

1. Behandlungskomplex (1a-1f) für eine anatomische Zone des menschlichen Körpers, wobei der Behandlungskomplex (1a-1f) wenigstens eine aktive Zone (2a-2f) zur Behandlung der anatomischen Zone umfasst, wobei die aktive Zone (2a-2f) mit Hauptanschlussmitteln (3a-3f, 3'a-3'f, 14a-14f), die beiderseits der aktiven Zone (2a-2f) vorgesehen und in der Lage sind, den Behandlungskomplex (1a-1f) mit der anatomischen Zone zu befestigen, gekoppelt ist, wobei die seitlichen Ränder des Behandlungskomplexes (1a-1f), wenigstens wenn der Behandlungskomplex (1a-1f) nicht getragen wird, getrennt und nicht untereinander verbunden sind,
**dadurch gekennzeichnet,**
**dass** die aktive Zone (2a-2f) wenigstens teilweise aus einem ersten Silikon hergestellt ist,
**dass** der Behandlungskomplex (1a-1f) von den Hauptanschlussmitteln (3a-3f, 3'a-3'f, 14a-14f) verschiedene sekundäre Anschlussmittel (7a-7f, 7'b, 7'c, 7'f, 16a-16f, 19) umfasst, die zwischen den Hauptanschlussmitteln (3a-3f, 3'a-3'f, 14a-14f) angeordnet sind und an die aktive Zone (2a-2f) gekoppelt sind, wobei die sekundären Anschlussmittel (7a-7f, 7'b, 7'c, 7'f, 16a-16f,19) wenigstens eine erste Haftzone umfassen, die wenigstens teilweise aus einem zweiten Silikon mit besseren Hafteigenschaften an die Haut als denjenigen des ersten Silikons hergestellt ist, wobei die erste Haftzone mit der Haut der anatomischen Zone zusammenwirken kann, um wenigstens zu dem Anhaften der aktiven Zone (2a-2f) auf der Haut beitragen zu können.

2. Behandlungskomplex (1a-1f) nach dem vorhergehenden Anspruch,
**dadurch gekennzeichnet, dass** die erste Haftzone wenigstens ein Lager (7a-7f, 7'b, 7'c, 7'f, 16a-16f, 19) mit einer bombierten Gestalt aufweist, das in Bezug auf die aktive Zone (2a-2f) eine erste Erhebung definiert.

3. Behandlungskomplex (1a-1f) nach dem vorhergehenden Anspruch,
**dadurch gekennzeichnet, dass** die erste Haftzone eine Mehrzahl von Lagern (7a-7f, 7'b, 7'c, 7'f) aus Silikon mit jeweils bombierter Gestalt aufweist, die in Bezug auf die aktive Zone (2a-2f) erste voneinander beabstandete Erhebungen definieren, die angeordnet sind, um zwischen einander ein freies Netzwerk vorzusehen, das die Zirkulation von Luft zwischen der anatomischen Zone und der aktiven Zone (2a-2f) zulassen kann, wenn der Behandlungskomplex (1a-1f) sich gegen die anatomische Zone abstützt.

4. Behandlungskomplex (1 a-1f) nach Anspruch 1, **dadurch gekennzeichnet, dass** die aktive Zone mit einer Öffnung (9a-9f) versehen ist, die in der Lage ist, einen Teil der anatomischen Zone aufzunehmen, dass die erste Haftzone einen die Öffnung (9a-9f) umgebenden Haltering (16a-16f) aus Silikon aufweist, wobei der Haltering (16a-16f) in Bezug auf die aktive Zone (2a-2f) eine erste Erhebung definiert.

5. Behandlungskomplex (1f) nach Anspruch 4, **dadurch gekennzeichnet, dass** die Öffnung (9f) kleinere Abmessungen aufweist als die Innenabmessungen des Halterings (16f), um eine Stützlippe (20) zu definieren, die von dem Haltering (16f) umgeben ist und die die Öffnung (9f) umgibt, wobei die Stützlippe (20) als Abstützung für einen entfernbaren Ring (21), der in dem Haltering (16f) untergebracht ist, dienen kann.

6. Behandlungskomplex (1f) nach dem vorhergehenden Anspruch, **dadurch gekennzeichnet, dass** die Stützlippe (20) durch einen Umbug (22) verlängert ist, der zu der Seite der Orthese (1f) gerichtet ist, die den Haltering (16f) aufweist, wobei der Umbug (22) mit der Stützlippe (20) und dem Haltering (6f) eine Kehle definiert, die in der Lage ist, den entfernbaren Ring (21) aufzunehmen und an seiner Halterung teilzuhaben.

7. Behandlungskomplex (1a-1f) nach einem der vorhergehenden Ansprüche, **dadurch gekennzeichnet, dass** er mit wenigstens einem aktiven Element (71d-71f) versehen ist, das wenigstens eine aktive Substanz umfasst, die in der Lage ist, zu der Haut zu diffundieren zum Sicherstellen der Verabreichung der aktiven Substanz auf transkutanem Weg.

8. Behandlungskomplex (1f) nach den Ansprüchen 5 und 7, **dadurch gekennzeichnet, dass** er wenigstens einen entfernbaren Ring (21) umfasst, der wenigstens eine aktive Substanz umfasst und das aktive Element (71 d-71f) bildet, wobei der entfernbare Ring (21) zwischen der Stützlippe (20) und dem Haltering (16f) eingesetzt ist.

9. Behandlungskomplex (1a-1f) nach einem der vorhergehenden Ansprüche, **dadurch gekennzeichnet, dass** zumindest die aktive Zone (2a-2f) frei von textilem Material ist.

10. Orthese (1a-1f) für eine anatomische Zone des menschlichen Körpers, wobei die Orthese (1a-1f) wenigstens ein Spannband umfasst, das elastisch deformierbar, spannbar und auf die anatomische Zone zur Sicherstellung seiner Halterung anbringbar ist, **dadurch gekennzeichnet, dass** sie in Gestalt eines Behandlungskomplexes (1a-1f) nach einem der vorhergehenden Ansprüche ausgebildet ist, wobei die Orthese (1a-1f) eine mittlere Zone (2a-2f) umfasst, die wenigstens teilweise die aktive Zone definiert und die auf einer ihrer Oberflächen mit wenigstens einem Teil der sekundären Anschlussmittel (7a-7f, 7'b, 7'c, 7'f, 16a-16f, 19) versehen ist.

11. Orthese (1a-1f) nach dem vorhergehenden Anspruch, **dadurch gekennzeichnet, dass** sie durch wenigstens zwei Halteabschnitte (4a-4f, 4'a-4'f) gebildet ist, die auf Höhe der mittleren Zone (2a-2f) übereinander gelegt und miteinander verbunden sind durch eine Stoßzone, die die seitlichen Ränder der mittleren Zone (2a-2f) der Orthese (1a-1f) begrenzt, wobei die Stoßzone eine geringere Breite aufweist als die der Halteabschnitte (4a-4f, 4'a-4'f).

12. Orthese (1f) nach Anspruch 11, **dadurch gekennzeichnet, dass** wenigstens einer der Halteabschnitte (4f, 4'f) wenigstens eine Verstärkung (17, 17'), wenigstens eine von der Verstärkung (17, 17') umgebene Ausnehmung und eine die Ausnehmung bedeckende Abdeckung (18, 18') aus Silikon umfasst.

13. Orthese (1f) nach Anspruch 2 und 10, **dadurch gekennzeichnet, dass** wenigstens einer der Halteabschnitte (4f, 4'f) mit wenigstens einer erhabenen Wulst (19) versehen ist, die gegen die anatomische Zone angelegt werden kann, wenn die Orthese (1f) getragen wird und die das Lager bildet, wobei die Wulst (19) ausgebildet ist zum Begrenzen einer wenigstens teilweise geschlossenen Zone, die wenigstens teilweise die anatomische Zone umgeben kann.

14. Orthese (1f) nach einem der Ansprüche 12 und 13, **dadurch gekennzeichnet, dass** wenigstens eines von Verstärkung (17, 17') und Wulst (19) eine Gestalt in Form einer Acht definiert, die wenigstens zwei geschlossene Zonen begrenzt, die dazu bestimmt sind, gegenüberliegend zu der anatomischen Zone angeordnet zu werden und mit den Hauptanschlussmitteln verbunden zu werden.

15. Orthese (1a-1f) nach Anspruch 10, **dadurch gekennzeichnet, dass** sie wenigstens zwei Paare von Verstärkungszonen (10a-10f, 10'a-10'f) aufweist, die voneinander beabstandet und seitlich versetzt in Bezug auf das Zentrum der mittleren Zone (2a-2f) sind, wobei jede Verstärkungszone (10a-10f, 10'a-10'f) eine höhere Steifigkeit aufweist als diejenige der mittleren Zone (2a-2f), wobei die Verstärkungszonen (10a-10f, 10'a-10'f) eines selben Paares gegenseitig gegenüberliegend angeordnet und miteinander durch wenigstens ein Versteifungselement (13a-13f), das elastisch deformierbar ist und eine höhere Steifigkeit aufweist als diejenige der mittleren Zone (2a-2f), verbunden sind.

16. Orthese (1a-1f) nach dem vorhergehenden Anspruch, **dadurch gekennzeichnet, dass** jede Verstärkungszone (10a-10f, 10'a-10'f) wenigstens ein Futteral (12a-12f) umfasst, das ein Ende des Versteifungselements (13a-13f) aufnehmen kann, wobei die Futterale (12a-12f) der Verstärkungszonen (10a-10f, 10'a-10'f) ein Paar bilden und einander gegenüberliegend vorgesehen sind, und dass das Versteifungselement (13a-13f) eine kleinere Länge aufweist als die Entfernung, die die entgegengesetzten Enden der Futterale (12a-12f) trennt, wodurch dieses zwischen den Enden zum Folgen der Winkeldeformation der Orthese (1a-1f) gleiten kann, ohne die Futterale (12a-12f) zu überschreiten.

## Claims

1. Treatment complex (1a-1f) for anatomic area of the human body, said treatment complex (1a-1f) comprising at least one active zone (2a-2f) for treating said anatomical area, said active zone (2a-2f) being coupled to main attachment means (3a-3f, 3'a-3'f, 14a-14f) provided on either side of said active zone (2a-2f) and adapted to fix the said treatment complex (1a-1f) to said anatomical area, the side edges of said treatment complex (1a-1f) being separated and unconnected at least when said treatment complex (1a-1f) is not brought, **characterized in that** said active zone (2a-2f) is at least partially made of a first silicone, **in that** said treatment complex (1a-1f) comprises second attachment means (7a-7f, 7'b, 7'c, 7'f, 16a-16f, 19), distinct from said main attachment means (3a-3f, 3'a-3'f, 14a-14f) disposed between said main attachment means (3a-3f, 3'a-3'f, 14a-14f) and coupled to said active zone (2a-2f), said second attachment means (7a-7f, 7'b, 7'c, 7'f, 16a-16f, 19) comprising at least a first adhesive area formed at least partially in a second silicone having characteristics of adhesion to the skin greater than those of said first silicone, said first adhesion area being adapted to cooperate with the skin of said anatomic area to at least contribute to the adhesion of said active zone (2a-2f) on said skin.

2. Treatment complex (1a-1f) according to the preceding claim, **characterized in that** said first adhesion area comprises at least one bearing (7a-7f, 7'b, 7'c, 7'f, 16a to 16f, 19) having a convex shape, defining with respect to said active zone (2a-2f) a first relief.

3. Treatment complex (1a-1f) according to the preceding claim, **characterized in that** said first adhesion area comprises a plurality of silicone bearings (7a-7f, 7'b, 7'c, 7'f) each having a convex shape, defining with respect to said active zone (2a-2f) first reliefs separated from each other arranged to form between them a free network suitable to allow the flow of air between said anatomical area and said active zone (2a-2f) when said treatment complex (1a-1f) bears against said anatomical area.

4. Treatment complex (1a-1f) according to claim 1, **characterized in that** said active zone is provided with an orifice (9a-9f) suitable for receiving a portion of the anatomical area, **in that** said first adhesion area comprises a maintaining ring (16a-16f) made of silicon surrounding said orifice (9a-9f), said maintaining ring (16a-16f) defining with respect to said active zone (2a-2f) a first relief.

5. Treatment complex (1f) according to claim 4, **characterized in that** said orifice (9f) has smaller dimensions than the inner dimensions of said maintaining ring (16f) so as to define a support lip (20) surrounded by said maintaining ring (16f) and surrounding said orifice (9f), said support lip (20) being adapted to provide support for a removable ring (21) accommodated in said maintaining ring (16f).

6. Treatment complex (1f) according to the preceding claim, **characterized in that** the support lip (20) is extended by a return (22) directed towards the side of said orthosis (1f) having said maintaining ring (16f), said return (22) defining, with said support lip (20) and said maintaining ring (16f), a groove adapted to receive said removable ring (21) and to contribute to its maintaining.

7. Treatment complex (1a-1f) according to any one of the preceding claims, **characterized in that** it is provided with at least one active element (71d-71f) comprising at least one active substance suitable of being diffused to said skin to assure the transcutaneous transmission of said active substance.

8. Treatment complex (1f) according to claims 5 and 7, **characterized in that** it comprises at least a removable ring (21) comprising at least one active substance and forming said active element (71d-71f), said removable ring (21) being inserted between said support lip (20) and said maintaining ring (16f).

9. Treatment complex (1a-1f) according to any one of the preceding claims, **characterized in that** at least said active area (2a-2f) is fabric material free.

10. Orthosis (1a-1f) for anatomic area of the human body, said orthosis (1a-1f) comprising at least one tension strip adapted to be deformed elastically, stretched and applied to said anatomical area in order to assure its maintaining, **characterized in that** the orthosis is in the form of a treatment complex (1a-1f) according to any one of the preceding claims, said orthosis (1a-1f) comprising a median region (2a-2f) defining at least in part said active area and provided on one of its surfaces at least a portion of said second attachment means (7a-7f, 7'b, 7'c, 7'f, 16a-16f, 19).

11. Orthosis (1a-1f) according to the preceding claim, **characterized in that** it is formed by at least two maintaining parts (4a-4f, 4'a-4'f) juxtaposed and connected together, at said median region (2a-2f), by a junction area defining the lateral edges of said median region (2a-2f) of said orthosis (1a-1f), said junction area having a smaller width than the width of said maintaining parts (4a-4f, 4'a-4'f).

12. Orthosis (1f) according to claim 11, **characterized in that** at least one of said maintaining parts (4f, 4'f) comprises at least one armature (17, 17'), at least one recess surrounded by said armature (17, 17') and a silicone voile (18, 18') overlying said recess.

13. Orthosis (1f) according to claim 10, **characterized in that** at least one of said maintaining parts (4f, 4'f) is provided with at least one protrusion (19) in relief adapted to be raised against anatomical area when said orthosis (1f) is brought and forming said bearing, said protrusion (19) being arranged to delimit one zone at least in part closed adapted to surround at least a portion of said anatomical area.

14. Orthosis (1f) according to one of claims 12 and 13, **characterized in that** at least one of said armature (17, 17'), protrusion (19), defines an eight shape defining at least two closed zones dedicated to be placed facing said anatomical area and connected to said main attachment means.

15. Orthosis (1a-1f) according to claim 10, **characterized in that** it comprises at least two pairs of reinforcing zones (10a-10f, 10'a-10'f), laterally spaced and offset relative to the center of said median region (2a-2f), each reinforcement zone (10a-10f, 10'a-10'f) having a rigidity greater than that of said median region (2a-2f), the reinforcement areas (10a-10f, 10'a-10'f) of a same pair being placed opposite one another and connected together by at least one stiffening element (13a-13f) elastically deformable and having a rigidity greater than that of said median region (2a-2f).

16. Orthosis (1a-1f) according to the preceding claim, **characterized in that** each reinforcement zone (10a-10f, 10'a-10'f) comprises at least a sheath (12a-12f) adapted to receive one end of said stiffening element (13a-13f), said sheaths (12a-12f) of the reinforcement zones (10a-10f, 10'a-10'f) constituting a pair provided opposite one another, **in that** said stiffening element (13a-13f) has a length less than the distance separating the opposed ends of said sheaths (12a-12f) allowing it to slide between said ends in order to follow the angular deformation of said orthosis (1a-1f), without exceeding said sheaths (12a-12f).
